Europäisches Patentamt

European Patent Office

Office européen des brevets

(11) Publication number: **0 252 645**
**A2**

# (12) EUROPEAN PATENT APPLICATION

(21) Application number: 87305591.7

(22) Date of filing: 23.06.87

(51) Int. Cl.4: **A61M 5/00**

(30) Priority: 09.07.86 US 883892
19.08.86 US 898156

(43) Date of publication of application:
**13.01.88 Bulletin 88/02**

(84) Designated Contracting States:
**AT BE CH DE ES FR GB GR IT LI LU NL SE**

(71) Applicant: ERBAMONT, INC.
Adria Laboratories 1266 Main Street
Stamford Connecticut 06092(US)

(72) Inventor: Strung, Mark Edward
5853 Harvest Oak
Dublin Ohio 43017(US)

(74) Representative: Bankes, Stephen C. D. et al
Baron & Warren 18 South End Kensington
London W8 5BU(GB)

(54) Method and apparatus for purging a syringe.

(57) A syringe purging device (100) comprises a liquidtight chamber (110) into which air and/or any excessive amount of a hazardous liquid, such as a chemotherapy drug, is ejected from a syringe (130) to overcome the health hazard created by openly purging syringes into the air or into a pad of gauze material. A sharp open end (136) of a hollow needle (134) of a syringe (130) is sealingly inserted into the liquidtight chamber (110), and the syringe (130) is activated to purge any air and/or excess liquid into the chamber (110). The liquidtight chamber (110) may be closed, with any liquid ejected thereinto being retained by absorption into a wad (112) of absorbent material contained within the chamber (110). After the syringe (130) is purged of any air and/or excess liquid, its needle (134) is then withdrawn from the chamber (110) such that the syringe (130) may be used for an injection.

FIG-4

## METHOD AND APPARATUS FOR PURGING A SYRINGE

The present invention relates generally to syringes for the injection of medicines into the body, and more particularly, to a method and apparatus for catching fluids ejected from a the needle of a syringe containing a hazardous or potentially hazardous liquid, such as a chemotherapy drug, as air and/or excess liquid is purged from the syringe.

The use of syringes for the hypodermic injection of medicines into the body through a hollow needle fixedly or detachedly forming a part of a syringe is commonplace in modern medicine. Preparation of a syringe for a hypodermic injection comprises drawing an approximate dosage of a medicine into a barrel of the syringe from a vial by means of a plunger sealingly fitted within the barrel of the syringe. Air is almost always included within the syringe barrel or the desired dosage exceeds that which is recommended. Accordingly, the syringe is held with the open end of its hollow needle extending generally upward and the plunger is inserted into the barrel to purge the air and/or excess medicine from the syringe prior to injection.

The purging spray from the needle is ordinarily harmless and may be simply squirted into the air or into a pad of gauze material. However, some fluids held by a syringe for injection or otherwise are hazardous or potentially hazardous, for example, chemotherapy drugs, for which the present invention is particularly applicable. When working with such fluids, the conventional purging operation described creates a dangerous health hazard to personnel utilizing the syringe. It is, thus, apparent that the need exists for a method and apparatus for purging a syringe to catch the spray which squirts from the open end of a hollow needle of a syringe when air and/or excessive portions of a hazardous or potentially hazardous liquid are ejected from the syringe to purge it.

In accordance with the present invention, the health hazard created by openly purging air and/or an excessive amount of a hazardous liquid, in particular a chemotherapy drug, from a syringe is overcome by catching the fluids purged from a syringe within a liquidtight chamber. Liquidtight chamber as used herein means that liquid cannot enter or exit the chamber other than being injected thereinto by means of the needle of a syringe which is sealingly inserted into the chamber.

According to one aspect of the present invention, a device for purging a syringe to catch fluids ejected therefrom comprises a liquidtight chamber for receiving the sharp open end of a hollow needle of a syringe therewithin such that the syringe can be activated to purge any air and/or excess liquid from the syringe into the chamber, and containment means associated with the chamber for preventing escape from the chamber of any liquid ejected thereinto.

In a first embodiment of the syringe purging device of the present invention, the chamber comprises a closed tubular member, one end of which is closed by stopper means for maintaining an airtight seal of the tubular member and permitting sealed entrance thereinto by a needle of a syringe puncturing the stopper means. In this embodiment, the containment means comprises a wad of absorbent material enclosed within the tubular member whereby any liquid injected into the chamber is absorbed by the absorbent material. The syringe purging device may further comprise flange means extending from the one end of the tubular member which is closed by the stopper means to facilitate handling and use of the device. Preferably, the tubular member of the syringe purging device of the present invention is formed from plastic and the stopper is formed from rubber, both of which are inert to liquids to be held by the device, for example, chemotherapy drugs.

In a second embodiment of the syringe purging device of the present invention, the chamber comprises a tubular member closed at one end by stopper means for maintaining an airtight seal of that end of the tubular member and permitting sealed entrance thereinto by a needle of a syringe puncturing the stopper device. The other end of the tubular member is closed except for communication with containment means which comprises a hydrophobic filter such that any air injected into the chamber or needed to replace liquid withdrawn therefrom is filtered by and passes through the hydrophobic filter out of or into the tubular member, and any liquid injected into the chamber is retained therein since it cannot pass through the hydrophobic filter.

A syringe may be purged using the second embodiment by inserting the open end of the hollow needle of a syringe into the chamber through the stopper and activating the syringe to eject any air and/or excess liquid therefrom. Air ejected from the syringe is filtered by and passes through the hydrophobic filter to exit the tubular member, while any excess liquid ejected from the syringe enters the chamber and is retained therein since it cannot pass through the hydrophobic filter. The needle is then withdrawn from the stopper such that it can be used for an injection. Alternately, the closed other end of the tubular member opposite to the stopper may include diaphragm means for permitting sealed extension of a needle of a syringe beyond the chamber by puncturing the diaphragm. If the device is operated in this manner, the syringe purging device remains on the needle while it is used for injection, typically into an intravenous tube or the like, and then is discarded with the syringe. Syringe purging devices of the present invention can be conveniently included in a needle cover, for example for a prefilled syringe, to facilitate handling and use of hazardous liquids, and particularly, chemotherapy drugs.

According to another aspect of the present invention, a method for purging a syringe to catch fluids ejected from a sharp open end of a hollow needle of a syringe containing a hazardous or potentially hazardous liquid as air and/or excess liquid is purged from the syringe comprises the steps of: inserting the sharp open end of a hollow needle of a syringe into a liquidtight chamber; manually operating the syringe to purge air and/or excess liquid from the syringe; retaining any ejected liquid within the chamber; and withdrawing the needle from the chamber or extending the needle beyond the chamber. In a method for purging a syringe in accordance with the present invention, the liquidtight chamber may be closed and the step of retaining any ejected liquid within the chamber then comprises absorbing any ejected liquid in absorbent material within the chamber Alternately, the liquid-tight chamber may be vented through a hydrophobic filter and then the step of retaining any ejected liquid within the chamber comprises blocking passage of liquid from the chamber through the hydrophobic filter.

It is, therefore, an object of the present invention to provide a method and apparatus for minimizing the health hazards associated with the use of a syringe containing hazardous or potentially hazardous liquids, particularly chemotherapy drugs; to provide an improved method and apparatus for catching fluids which squirt from the needle of a syring containing a hazardous of potentially hazardous liquid as air and/or excessive amounts of the liquid are purged from the syringe; and to provide a method and apparatus for catching fluids ejected from a sharp open end of a hollow needle of a syringe containing hazardous or potentially hazardous liquids, in particular chemotherapy drugs, as air and/or excess liquid is purged from the syringe by catching the fluids purged from the syringe within a liquidtight chamber.

Other objects and advantages of the invention will be apparent from the following description, the accompanying drawings and the appended claims.

In order that the invention may be more readily understood, reference will now be made to the accompanying drawings, in which:

Fig. I is a side view of a first embodiment of a syringe purging device in accordance with the present invention.

Fig. 2 is a top view of the syringe purging device of Fig. I showing the needle entryway of the device.

Figs. 3 and 4 are partially sectioned side views of the syringe purging device of Figs. I and 2 showing operation of the device for purging a syringe of air and/or excess liquid.

Figs. 5-9 show alternate embodiments of syringe purging devices in accordance with the present invention and the usage of those embodiments.

Different embodiments of a syringe purging device in accordance with the present invention are shown in the drawing figures wherein identical or corresponding parts have been identified by like reference numerals throughout. A first embodiment of a syringe purging device I00 in accordance with the present invention is shown in drawing Figs. I-4. The device I00 comprises a closed tubular member I02, one end I04 of which is closed by stopper means comprising a resilient stopper I06 fixedly secured within the tubular member I02. The other end I08 of the tubular member I02 preferably is formed of the same material and is contiguous with the remainder of the closed tubular member I02. The tubular member I02 thus defines a liquidtight chamber II0 which extends between the closed ends I04 and I08 of the closed tubular member I02.

While other materials can be used, preferably, the tubular member I02 is formed from translucent plastic and the stopper I08 is formed from rubber. The use of translucent plastic permits a clear view of the operation of the syringe purging device I00 to encourage proper and safe utilization. Whatever materials are used to construct a syringe purging device in accordance with the present invention, it is to be understood that they must be inert to the liquids to be used by the syringe, for example, chemotherapy drugs for which the syringe purging device is particularly useful.

The stopper I06 comprises a generally cylindrical body which includes an opening I06A positioned toward the inside of the closed tubular member I02, i.e., the chamber II0, and a concave outer wall I06B which is formed to be penetrated by the needle of a syringe with no coring action. The concave outer wall I06B is of an appropriate thickness such that it can be readily punctured by the sharp open end of a hollow needle of a syringe, and provide sealing engagement with the needle to thereby maintain the chamber II0 liquidtight yet in communication with the contents of a syringe via its hollow needle. The stopper I06 must also provide good resealability such that the fluids con-

tained within the chamber 110 will not leak and will be retained therein for an indefinite period of time after the syringe needle is withdrawn from the stopper 106.

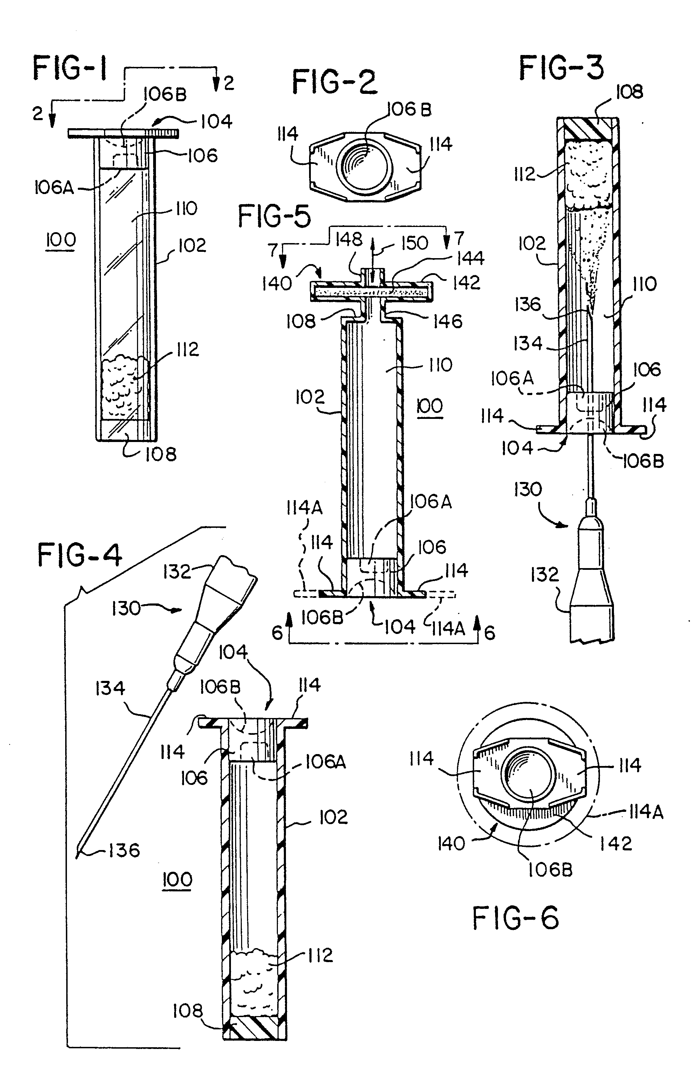

Fluid containment means is associated with the chamber 110 for preventing the escape of any liquid ejected into the chamber 110. In the first illustrative embodiment of the present invention shown in Figs. 1-4, the containment means comprises a wad 112 of absorbent material, such as cotton, enclosed within the tubular member 102 such that any liquid injected into the chamber 110 is absorbed by the absorbent material. The wad 112 of absorbent material can also contain a deactivating substance to neutralize the hazardous characteristics of any liquid absorbed by the absorbent material. In the case of chemotherapy drugs, bleach is an example of one such deactivating substance.

To faciliate handling and use of the syringe purging device 100, flange means extend from the end 104 of the tubular member 102 which is closed by the stopper 106. It is noted that the flange means can be of various shapes. For example, the flange means may range from diametrically opposed planar flanges 114 shown in solid line drawings in the figures to an expanded circular flange 114A shown in dotted-line drawings in Figs. 5-7. Expanded flanges, such as the suggested circular flange 114A, can also serve to shield and protect the user from accidental needle pricks as a syringe needle is inserted into the device 100.

Operation of the first embodiment of a syringe purging device in accordance with the present invention as shown in Figs. 1-4 will now be described with reference particularly to Figs. 3 and 4. A syringe 130 comprises a barrel 132 with a movable plunger (not shown) positioned therein for drawing fluids into the barrel 132 and ejecting fluids therefrom through a hollow needle 134 of the syringe 130, as is well known in the art. The sharp open end 136 of the needle 134 is inserted into a vial or dispensing container (not shown) which contains medicine to be injected by the syringe 130. The plunger is inserted entirely into the barrel 132 and then withdrawn to draw medicine from the vial into the barrel 132 for a hypodermic injection.

As is almost always the case, air will also be contained within the barrel 132 and/or the amount of medicine or other liquid desired to be drawn into the barrel 132 will exceed by a small amount the necessary quantity. In that event, the air and/or small amount of excess liquid contained within the barrel 132 must be purged prior to use of the syringe 130. As previously noted, usually the spray from the needle 134 is harmless and may be simply squirted into the air or into a pad of gauze material. However, some fluids held by the syringe 130 may be hazardous or potentially hazardous, for example, a chemotherapy drug, such that performing the purging operation in the open air or into a pad of gauze material may be hazardous to personnel utilizing the syringe. In such cases, the syringe purging device 100 of the present invention is conveniently utilized as follows.

The sharp open end 136 of the needle 134 is inserted into the chamber 110 by engaging the concave outer wall 106B of the stopper 106 preferably near its center where it is forced to puncture the stopper 106 for sealed insertion into the chamber 110 as shown in Fig. 3. The syringe 130 is then activated by forcing its plunger into the barrel 132, with the needle 134 being in a generally upward direction such that air within the barrel 132 is adjacent to and will be ejected through the needle 134 into the chamber 110 to purge the air from the barrel 132. This purging operation is shown in Fig. 3 wherein the air is injected into the chamber 110. Some portion of the liquid contained within the barrel 132 is also ejected into the chamber 110 either inadvertently with the air or to eliminate excess liquid from the barrel 132 such that a desired quantity of the liquid is contained within the barrel 132. It is noted that syringe purging devices of the present invention are sterilized such that any liquid ejected from the syringe can be redrawn into the syringe if an excessive amount of the liquid is inadvertently ejected into the chamber 110.

Once the air and/or any excess liquid has been purged from the barrel 132, the syringe 130 and syringe purging device 100 are inverted to the position shown in Figs. 1 and 4 such that the needle is in a generally downward direction, and any liquid ejected into the chamber 110 with insufficient force to engage the wad 112 of absorbent material enclosed within the end of the chamber 110 will flow toward that end of the chamber where it too is absorbed. The syringe needle 134 can then be withdrawn from the stopper 106 such that it is ready for use as shown in Fig. 4. The absorption of any liquid ejected into the chamber 110 by the absorbent material and the resealability characteristics of the stopper 106 ensure that any liquid ejected from the syringe 130 remains within the chamber 110 as the needle 134 is withdrawn from the stopper 106.

Alternate embodiments of a syringe purging device in accordance with the present invention and the operation of those embodiments is shown in Figs. 5-9. Figs. 5-7 show a partially sectioned side view, bottom view and top view, respectively, of a syringe purging device 100 which comprises a tubular member 102 closed at one end 104 by stopper means comprising a resilient stopper 106 fixedly secured in the one end 104 for maintaining

an airtight seal of that end of the tubular member 102. The stopper 106 permits sealed entrance into a chamber 110 defined by the tubular member 102 by a needle of a syringe puncturing the stopper 106.

The other end 108 of the tubular member 102 is closed except for communication with containment means which, in this embodiment of the syringe purging device 100, comprises a hydrophobic filter 140. While the hydrophobic filter may take any of a number of forms, in the illustrated embodiment, it comprises a circular disc-like housing 142 for holding a hydrophobic filter element 144 therewithin such that air entering the housing 142 through passages 146 and 148 must pass through the hydrophobic filter element 144. The passage 146 provides communication between the chamber 110 defined by the tubular member 102 and the hydrophobic filter 140. Air may pass through the hydrophobic filter 140 through the passages 146 and 148 as indicated by the arrow 150 such that air injected into the chamber 110 or needed to replace liquid withdrawn therefrom (for example, if excess liquid is inadvertently injected into the chamber 110) is filtered by and passes through the hydrophobic filter 140, while any liquid injected into the chamber 110 is retained therein since it cannot pass through the hydrophobic filter 140.

A syringe may be purged using the embodiment shown in Figs. 5-7 by inserting the open end of a hollow needle of a syringe into the chamber 110 through the stopper 106 and activating the syringe to eject any air and/or excess liquid therefrom. Air ejected from the syringe is filtered by and passes through the hydrophobic filter 140 to exit the tubular member 102, while any excess liquid ejected from the syringe enters the chamber and is retained therein since it cannot pass through the hydrophobic filter 140. The needle is then withdrawn from the stopper 106 such that it can be used for an injection.

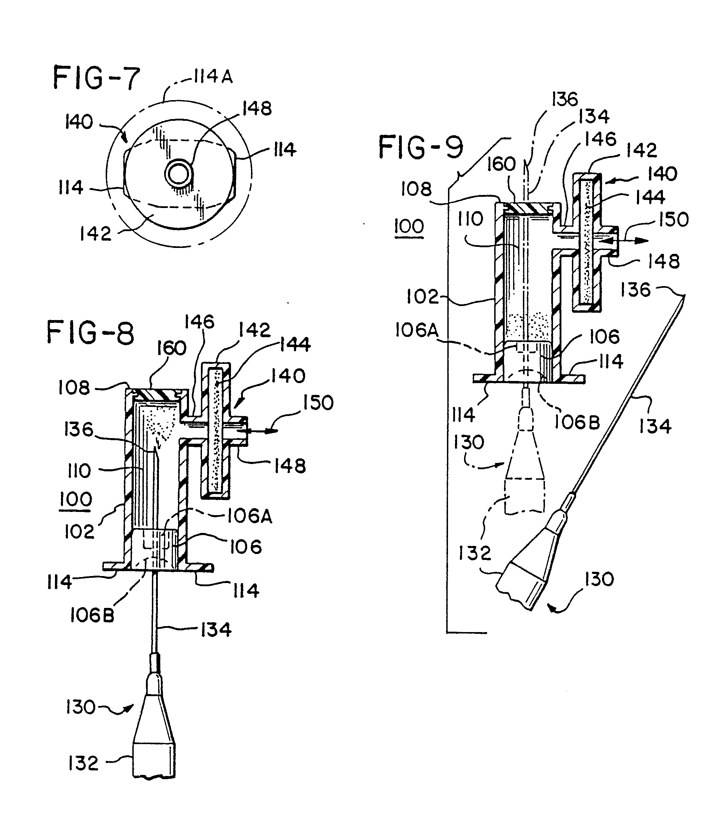

An alternate of the embodiment of the syringe purging device 100 of Figs. 5-7 is shown in Figs. 8 and 9. In this embodiment, the liquidtight chamber 110 is vented through a hydrophobic filter 140 which is placed in communication with the chamber 110 on the side of the tubular member 102. The closed other end of the tubular member 102 opposite to the stopper 106 may then include diaphragm means 160 for permitting sealed extension of a needle of a syringe beyond the chamber 110 by puncturing the diaphragm 160. If the device 100 of Figs. 8 and 9 is used in this manner, the device 100 remains on the needle as it is used for injection, typically into an intravenous tube or the like, and then the device 100 is discarded with the syringe.

A syringe may be purged using this embodiment by inserting the sharp open end 136 of the hollow needle 134 of the syringe 130 into the chamber 110 through the stopper 106 and activating the syringe to eject any air and/or excess liquid therefrom. Air ejected from the syringe 130 is filtered by and passes through the hydrophobic filter 140 to exit the tubular member 102, while any excess liquid ejected from the syringe 130 enters the chamber 110 and is retained therein since it cannot pass through the hydrophobic filter 140. The needle may then be withdrawn from the stopper as shown by the solid line drawing in Fig. 9 such that it can be used for an injection. Alternately, the sharp open end 136 of the needle 134 can be extended beyond the chamber 110 by puncturing the diaphragm 160 as shown by the dotted line drawing in Fig. 9.

It is noted that the stoppers 106 of syringe purging devices in accordance with the present invention could be diaphragms or the like which would close the devices as described, yet permit sealed penetration of a syringe needle therethrough. The syringe purging devices of the present invention can also be conveniently included in a needle cover. If such a needle cover is used on a prefilled syringe, the syringe can be purged and ready for administration of an injection before the cover is removed. In syringes which are not prefilled, the syringe needle can be reinserted into the previously removed needle cover to perform the purging operation.

While the methods herein described and the forms of apparatus for carrying these methods into effect constitute preferred embodiments of this invention, it is to be understood that the invention is not limited to these precise methods and forms of apparatus, and that changes may be made in either without departing from the scope of the invention as defined in the appended claims.

**Claims**

1. A syringe purging device (100) for catching fluids ejected from a sharp open end (136) of a hollow needle (134) of a syringe (130) containing a hazardous or potentially hazardous liquid as air and/or excess liquid is purged from the syringe (130), said device comprising a liquidtight chamber (110) for receiving the sharp open end (136) of a hollow needle (134) of a syringe (130) therewithin, and containment means (112,140) associated with said chamber (110) for preventing the escape from said chamber (110) of any liquid ejected thereinto whereby a syringe (130) may be purged by inserting the needle (134) of the syringe (130) into said chamber (110) and activating the syringe (130) to

eject any air and/or excess liquid therefrom with any excess liquid being retained within said chamber (110) as the needle (134) is withdrawn from or extended beyond said chamber (110).

2. A syringe purging device (100) as claimed in claim 1 wherein said chamber (110) comprises a closed tubular member (102), one end (104) of said tubular member (102) being closed by stopper means (106) for maintaining an airtight seal of said tubular member (102) and permitting sealed entrance thereinto by a needle (134) of a syringe (130) puncturing said stopper means (106), and said containment means comprises a wad (112) of absorbent material enclosed within said tubular member (102) whereby any liquid ejected into said chamber (110) is absorbed by said absorbent material.

3. A syringe purging device (100) as claimed in claim 2 wherein said wad (112) of absorbent material contains a deactivating substance to neutralize the hazardous characteristics of any liquid ejected into said chamber (110).

4. A syringe purging device (100) as claimed in claim 3 further comprising flange means (114) extending from said one end (104) of said tubular member (102) to facilitate handling and use of said device.

5. A syringe purging device (100) as claimed in claim 4 wherein said tubular member (102) is formed from plastic and said stopper means (106) is formed from rubber.

6. A syringe purging device (100) as claimed in claim 1 wherein said chamber (110) comprises a tubular member (102) closed at one end (104) by stopper means (106) for maintaining an airtight seal of said one end (104) of said tubular member (102) and permitting sealed entrance thereinto by a needle (134) of a syringe (130) puncturing said stopper means (106), the other end (108) of said tubular member (102) being closed except for communication with said containment means which comprises a hydrophobic filter (140) whereby any air ejected into said chamber (110) is filtered by and passes through said hydrophobic filter (140) out of said tubular member (102) and any liquid ejected into said chamber (110) is retained therein since it cannot pass through said hydrophobic filter (140).

7. A syringe purging device (100) as claimed in claim 6 wherein the closed other end (108) of said tubular member (102) opposite to said stopper means (106) includes diaphragm means (160) for permitting sealed extension of a needle (134) of a syringe (130) beyond said chamber (110) by puncturing said diaphragm means (160).

8. A syringe purging device (100) as claimed in claim 7 further comprising flange means (114) extending from said one end (104) of said tubular member (102) to facilitate handling and use of said device.

9. A syringe purging device (100) as claimed in claim 8 wherein said tubular member (102) is formed from plastic and said stopper means (106) is formed from rubber.

10. A method for catching fluids ejected from a sharp open end (136) of a hollow needle (134) of a syringe (130) containing a hazardous or potentially hazardous liquid as air and/or excess liquid is purged from the syringe (130) comprising the steps of:

inserting the sharp open end (136) of a hollow needle (134) of a syringe (130) into a liquidtight chamber (110);

manually operating the syringe (130) to purge air and/or excess liquid from the syringe (130);

retaining any ejected liquid within said chamber (110); and

withdrawing the needle (134) from said chamber (110).

11. A method for purging a syringe (130) as claimed in claim 10 wherein said liquidtight chamber (110) is closed and the step of retaining any ejected liquid within said chamber (110) comprises absorbing any ejected liquid in absorbent material (112) within said chamber (110).

12. A method for purging a syringe (130) as claimed in claim 11 further comprising the step of introducing a deactivating substance into said absorbent material (112) whereby the hazardous characteristics of any liquid ejected into said liquidtight chamber (110) and absorbed by said absorbent material (112) are neutralized.

13. A method for purging a syringe (130) as claimed in claim 10 wherein said liquidtight chamber (110) is vented through a hydrophobic filter (140) and the step of retaining any ejected liquid within said chamber (110) comprises blocking passage of liquid from said chamber (110) through said hydrophobic filter (140).

14. A method for catching fluids ejected from a sharp open end (136) of a hollow needle (134) of a syringe (130) containing a hazardous or potentially hazardous liquid as air and/or excess liquid is purged from the syringe (130) comprising the steps of:

inserting the sharp open end (136) of a hollow needle (134) of a syringe (130) into a liquidtight chamber (110);

manually operating the syringe (130) to purge air and/or excess liquid from the syringe (130);

retaining any ejected liquid within said chamber (110); and

extending the needle (134) beyond said chamber (110).

15. A method for purging a syringe (130) as claimed in claim 14 wherein said liquidtight chamber (110) is vented through a hydrophobic filter (140) and the step of retaining any ejected liquid within

said chamber (110) comprises blocking passage of liquid from said chamber (110) through said hydrophobic filter (140).

FIG-1

FIG-2

FIG-3

FIG-5

FIG-4

FIG-6

FIG-7

FIG-8

FIG-9